# EUROPEAN PATENT SPECIFICATION

(11) **EP 2 077 134 B1**
(45) Date of publication and mention of the grant of the patent: **01.12.2010**
(21) Application number: 08164267.0
(22) Date of filing: 12.09.2008
(51) Int. Cl.: A61M 25/06, A61M 39/06

(54) **Safety I.V. catheter with in-built device to prevent blood back flow**
IV-Sicherheitskatheter mit integrierter Vorrichtung zur Verhinderung von Blutrückfluss
Cathéter I.V. sécurisé avec dispositif intégré pour empêcher le reflux sanguin

(30) Priority: 03.01.2008 IN KA00232008
(43) Date of publication of application: 08.07.2009
(73) Proprietor: Eastern Medikit Ltd., 110 007 Delhi (IN)
(72) Inventor: Narang Karun, Raj, 110 007, Delhi (IN); Paul, Sanjeev, 110 007, Delhi (IN)
(74) Representative: Harrison, Robert John

(56) References cited:
- WO-A-96/40360
- FR-A- 2 447 201
- US-A- 4 245 635
- US-A- 4 950 254
- US-A- 5 263 943
- US-A1- 2005 283 123

## Description

The following specification particularly describes the nature of this invention and the manner in which it is to be performed:
This invention relates to a I.V. Catheter and, in particular, to an I.V. Catheter with an inbuilt device to prevent Blood Back Flow. In particular, the intravenous catheter device for the invention has been provided to prevent back flow of blood after cannulation and at the time of withdrawal of needle.

### BACKGROUND ART

It is well known that when cannulation is done i.e. when needle is inserted in the vein of a patient, blood comes out into the plastic tubing in which the needle is fitted. The purpose of cannulation is to leave the plastic tubing inside the vein and the needle is withdrawn and disposed off. In this process, blood rushes into the tube and body of the I.V. Catheter and even comes out.

To prevent this, paramedical staff presses the vein to avoid rush of blood. This method is too primitive, uncertain and ineffective in many cases.

US patent No. 4,245,635 titled "Catheter assembly for intermittent intravenous use" assigned to Jelco Laboratories discloses a catheter assembly having an elongated hollow tube and a hub connected to the elongated hollow tube. The hub has an interior cavity communicating with a lumen of the elongated hollow tube and also includes an aperture communicating with the cavity. An operable valve includes a closure portion located in the cavity. The operable valve is adapted to close the interior entrance of the aperture under the influence of fluid flowing into the cavity from the lumen after a needle is withdrawn. The operable valve is a ball shaped or cone shaped element to seal the aperture.

A US patent No. 4,950,254 titled "Valve means for enteral therapy administration set" and assigned to Corpak Inc. discloses a one way valve for incorporation into a fluid administration set for enteral therapy. The valve responds to backflow pressure and arrests retrograde movement of fluid into the set, thereby avoiding rupturing of a tubing in the set.

A US patent No. 5,263,943 titled "Valved intravenous needle assembly" discloses a needle assembly including an elongate first cylindrical cavity with a first and second end, with the second end formed with a conical recess. The conical recess including a through-extending bore in communication with the recess is directed into a second cavity. The needle assembly prevents reverse fluid flow from the first cylindrical body into the second cylindrical body.

A French patent application No. 2,447,201 titled "Catheter assembly for intermittent intravenous medicament delivery" and assigned to Jelco Laboratories discloses a catheter assembly having an elongated hollow tube and a hub connected to the elongated hollow tube. The hub has an interior cavity communicating with a lumen of the elongated hollow tube and also includes an aperture communicating with the cavity. A blocking device is contained in the cavity which is adapted to block the entrance to the lumen after a needle is withdrawn from the catheter assembly. The blocking device may be a ball which is moveable within the cavity so that fluids injected into the cavity will be permitted to enter into the lumen.

### OBJECTS OF THE INVENTION

The purpose of this invention is to provide an I.V. Catheter which would by its own mechanism prevent such rush of blood after cannulation.

### SUMMARY OF THE INVENTION

Thus according to the basic aspect of the present invention there is provided an intravenous (I.V.) Catheter with inbuilt device to prevent blood back flow comprising:
The I.V. Catheter comprises of a body, a needle hub capable of holding the needle in a manner that the non sharp end of the needle is fitted inside the needle hub and sharp end is used for cannulation. The needle passes into the plastic tubing for the purpose of cannulation.

The details of the invention, its objects and advantages are explained hereunder in greater detail in relation to non-limiting exemplary illustration as per the following accompanying figures:

### BRIEF DESCRIPTION OF THE ACCOMPANYING FIGURES

Figure 1 illustrates an embodiment of the intravenous (I.V.) Catheter.

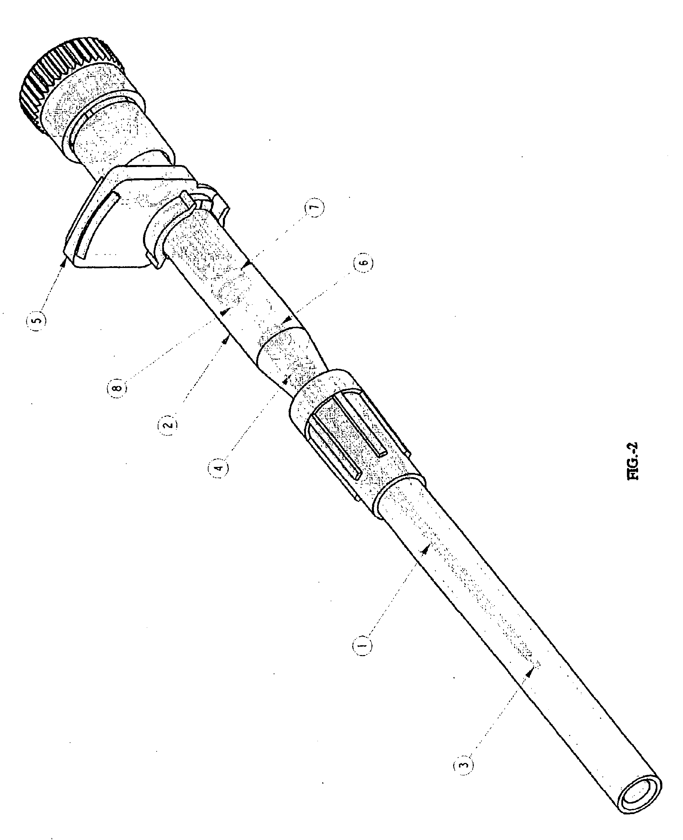

Figure 2 is the complete intravenous (I.V.) Catheter wherein the individual parts have been numbered as below:
   1. Portion marked as I is plastic tubing.
   2. Portion marked as 2 is body of the I.V. Catheter.
   3. Portion marked as 3 is needle.
   4. Portion marked as 4 is Catheter holder.
   5. Portion marked as 5 is needle hub.
   6. Portion marked as 6 is washer Numbered as I.
   7. Portion marked as 7 is washer Numbered as II.
   8. Portion marked as 8 is ball.

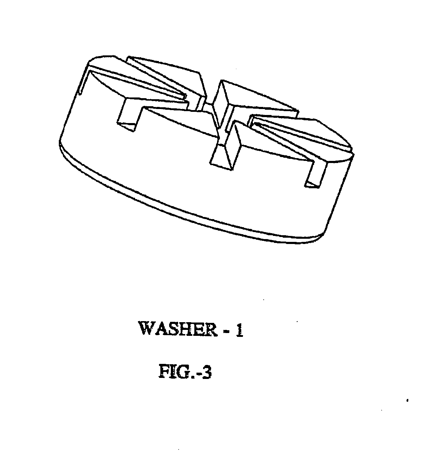

Figure 3 is an illustration of the washer Numbered as I having deep grooves with hole in the Centre.

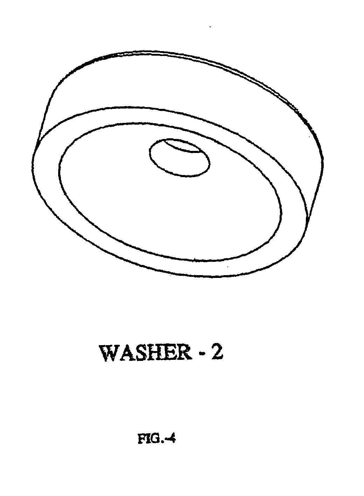

Figure 4 is washer Number II.

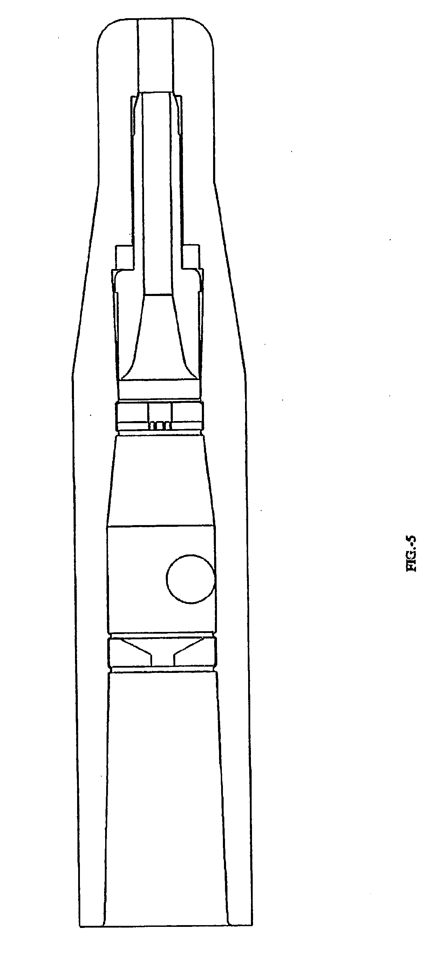

Figure 5 is washer Number I and Number II fitted inside the body of the I.V. Catheter.

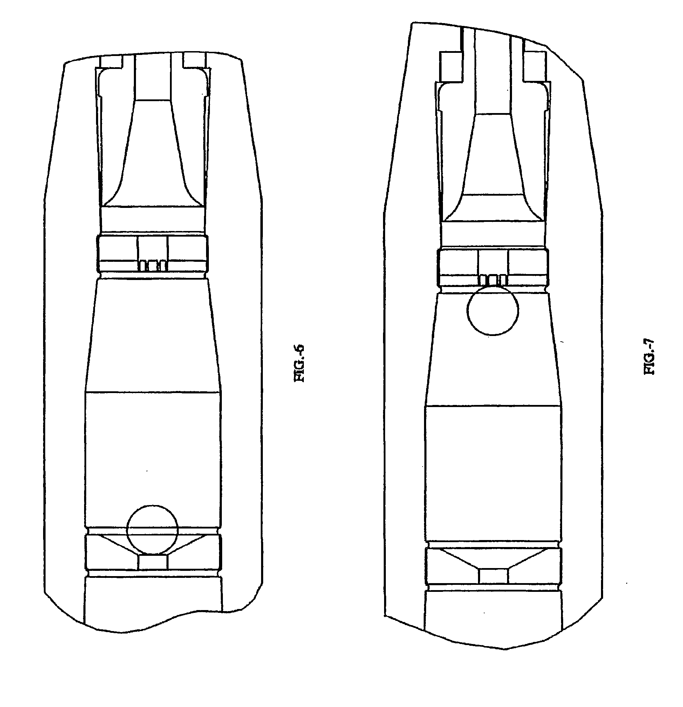

Figures 6 and 7 show the working principle of the invention.

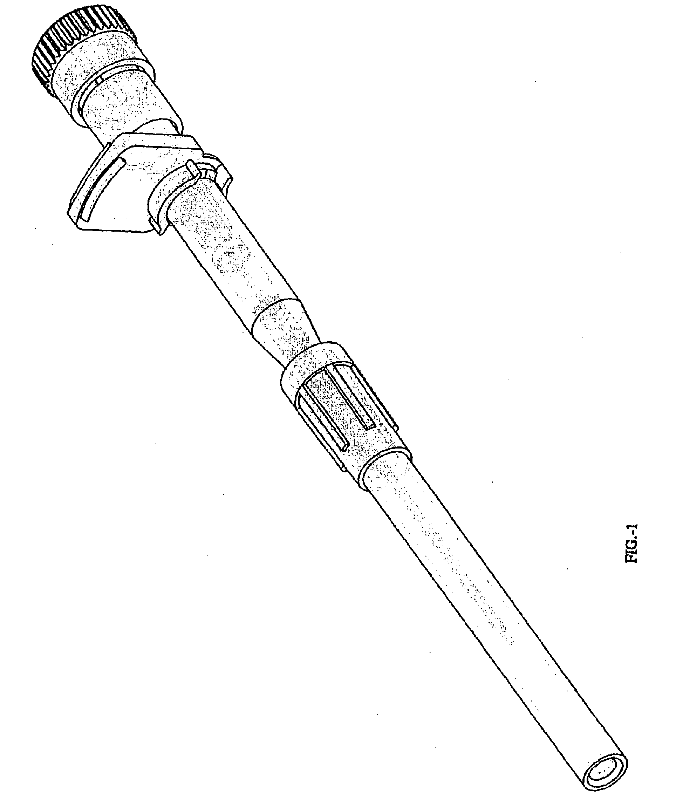

Reference is first invited to accompanying figure 1, which shows the intravenous i.v. Catheter device in accordance with the present invention wherein the product I.V. Catheter has two washers and a ball positioned below the needle. The needle is fitted in the needle hub and passes through the centric hole in the two washers.

Reference is now invited to Figures 3 and 4, which illustrate the washer Number I and washer Number II respectively.

Reference is now invited to accompanying figure 5.

Figure 5 shows the washer I and II fitted inside the body of the I.V. Catheter. Washer Number II which is plain and has a hole in the centre is positioned in the body of catheter at the end closer to the needle hub. Washer Number I which has deep grooves and has a hole in the centre is positioned in the body of the catheter at the end closer to plastic tubing fitted to the body of the I.V. Catheter.

Such a provision of the intravenous (I.V.) Catheter device therefore achieves the required safety in IV cannulation and adapted to protect the user against needle stick injuries.

The method of application and use of the above safety intravenous (I.V.) Catheter of the invention is further detailed hereunder:-
Thus in use of the catheter device, initially the site of the catheter insertion on the patient's body is cleaned using alcohol wipes. Thereafter, the skin is punctured by using the needle tip which is inside a thin plastic tube. The needle tip is moved forward to puncture the vein. Immediately blood flash back is noticed in the blood collection chamber.

The blood is thus unable to come out from the body and thus spillage is avoided. This is an added safety feature making the user safe from the blood of the patient. Now, the fluid is administered. The fluid is administered through the body of the cannula. The pressure of fluid would displace the ball stationed at centric hole of washer Number II. The pressure of the fluid would take the ball to the centric hole of the washer Number No. I. However, this washer is provided with grooves in the nature of lanes. The fluid would push through the lanes and bypass the ball from distance provided between the ball and hole (by method of grooves).

Subsequently, the needle withdrawing action is initiated by withdrawing the needle out from the cannula and leaving the plastic tubing inside the body. The body of the catheter remains outside. In this invention, it is proposed that when the blood will rush from the vein into the body of I.V. Catheter, the blood by its force will push the ball stationed between the washers would be pushed towards washer Number II and block the hole so that the blood became impassable through that hole. The dimension of the ball is such that it is more than the circumference of the centric hole of the two washers.

Advantageously, the above (I.V.) Catheter is adapted to function as safe device to protect the user from exposure to blood during cannulation. The above (I.V.) Catheter device can be obtained of simple materials including polypropylene, low density poly ethylene, stainless steel, Fluoropolymer/ Poly urathene.

The body, needle hub, flash back, are made of polypropylene. The needle and ball are made of stainless steel. However, the ball can be made of plastic/ titanium.

## Claims

1. An intravenous (I.V.) Catheter device with a retractable needle comprising:
An inbuilt device to prevent blood back flow and where there is a body (2) of the I.V. Catheter, a needle hub (5), a ball (8) **characterised by**:
a washer Number I (6) and a washer Number II (7), wherein the ball (8) is placed between the washer Number I (6) and the washer Number II (7) and wherein the retractable needle passes through a centric hole of the washer Number I (6) and a centric hole of the washer Number II (7) into a plastic tubing (1) and on withdrawal of the needle after cannulation, a flow of blood pushes the ball (8) to the centric hole of the washer Number II (7) and blocks flow and wherein conversely a fluid administered into the body (2) of the I.V catheter pushes the ball (8) to the centric hole of the washer Number I (6) and fluid get passage through grooves present in the washer Number I(6).

2. The intravenous (I.V.) Catheter device as claimed in claim 1, wherein the ball (8) is positioned below the needle.

3. The intravenous (I.V.) catheter as claimed in claim 1, wherein the washer Number I (6) is positioned at an end closer to the needle hub (5).

4. The intravenous (I.V.) catheter device as claimed in claim 1, wherein the washer Number II (7) is positioned in the body (2) of the I.V catheter at an end, closer to the plastic tubing (1) fitted to the body (2) of the I.V. Catheter.

5. The intravenous (I.V.) Catheter device as claimed in claim 1, wherein the ball (8) is larger then the outer circumference of the centric hole of the washer Number I (6) and the centric hole of the washer Number II (7).

6. The intravenous (I.V.) Catheter device as claimed in claim 1, wherein the ball (8) comprises one of stainless steel, titanium or plastic.

7. The intravenous (I.V.) Catheter device as claimed in claim 1, wherein the needle comprises stainless steel.

8. The intravenous (I.V.) Catheter device as claimed in claim 1, wherein the body (2) and/or the needle hub (5) of the I.V. Catheter comprise polypropylene.

## Patentansprüche

1. Intravenöse (I.V.) Kathetervorrichtung mit einer einfahrbaren Nadel, umfassend: eine eingebaute Vorrichtung um einen Blutrückfluss zu verhindern und weiters umfassend einen Körper (2) des I.V. Katheters, eine Nadelhalterung (5), eine Kugel (8), **dadurch gekennzeichnet**:
eine Scheibe Nummer I (6) und eine Scheibe Nummer II (7), wobei die Kugel (8) zwischen der Scheibe Nummer I (6) und der Scheibe Nummer II (7) angeordnet ist, und wobei die einfahrbare Nadel durch ein zentrisches Loch der Scheibe Nummer I (6) und einem zentrischem Loch der Scheibe Nummer II (7) in eine plastische Röhre (1) hindurch steht, und bei einem zurückziehen der Nadel nach einer Kanülierung ein Blutfluss die Kugel (8) in das zentrische Loch der Scheibe II (7) drückt und den Fluss blockiert, und wobei im Gegensatz dazu eine Flüssigkeit die in den Körper (2) des I.V. Katheters appliziert wird, die Kugel (8) zum zentrischen Loch der Scheibe I (6) drückt und den Weg für das Fluid durch Nuten in der Scheibe Nummer I (6) freigibt.

2. Intravenöse (I.V.) Kathetervorrichtung gemäß Anspruch 1, wobei die Kugel (8) unter der Nadel positioniert ist.

3. Intravenöse (I.V.) Kathetervorrichtung gemäß Anspruch 1, wobei die Scheibe Nummer I (6) an einem Ende, näher an der Nadelhalterung (5) gelegen, positioniert ist.

4. Intravenöse (I.V.) Kathetervorrichtung gemäß Anspruch 1, wobei die Scheibe Nummer II (7) im Körper (2) des I.V. Katheters an einem Ende, näher zur plastischen Röhre (1), welche an dem Körper (2) des I.V. Katheters angebracht ist, positioniert ist.

5. Intravenöse (I.V.) Kathetervorrichtung gemäß Anspruch 1, wobei die Kugel (8) größer ist als der äußere Umfang des zentrischen Lochs der Scheibe Nummer I (6) und des zentrischen Lochs der Scheibe Nummer II (7).

6. Intravenöse (I.V.) Kathetervorrichtung gemäß Anspruch 1, wobei die Kugel (8) einen von rostfreiem Stahl, Titan oder Plastik umfasst.

7. Intravenöse (I.V.) Kathetervorrichtung gemäß Anspruch 1, wobei die Nadel rostfreiem Stahl umfasst.

8. Intravenöse (I.V.) Kathetervorrichtung gemäß Anspruch 1, wobei der Körper (2) und/oder die Nadelhalterung (5) des I.V. Katheters Polypropylen umfassen.

## Revendications

1. Dispositif de cathéter intraveineux (I.V) avec une aiguille rétractable, comprenant :
un dispositif intégré pour empêcher le reflux sanguin, et dans lequel il y a un corps (2) du cathéter I.V, un pavillon d'aiguille (5), une boule (8), **caractérisé par** :
une rondelle numéro I (6) et une rondelle II (7), dans lequel la boule (8) est placée entre la rondelle numéro I (6) et la rondelle numéro II (7), et dans lequel l'aiguille rétractable passe à travers un trou centrique de la rondelle numéro I (6) et un trou centrique de la rondelle numéro II (7), et à l'intérieur d'un tube en plastique (1), et lors du retrait de l'aiguille après cannulation, un flux de sang pousse la boule (8) vers le trou centrique de la rondelle numéro II (7) et bloque le flux, et dans lequel, à l'inverse, un fluide administré dans le corps (2) du cathéter I.V. pousse la boule (8) vers le trou centrique de la rondelle numéro I (6) et le fluide obtient le passage à travers des rainures présentes dans la rondelle I (6).

2. Dispositif de cathéter intraveineux (I.V.) selon la revendication 1, dans laquelle la boule (8) est positionnée sous l'aiguille.

3. Cathéter intraveineux (I.V.) selon la revendication 1, dans lequel la rondelle numéro I (6) est positionnée à une extrémité plus proche du pavillon d'aiguille (5).

4. Dispositif de cathéter intraveineux (I.V.) selon la revendication 1, dans lequel la rondelle numéro II (7) est positionnée dans le corps (2) du cathéter I.V. à une extrémité, plus proche du tube en plastique (1) ajusté dans le corps (2) du cathéter (I.V.).

5. Dispositif de cathéter intraveineux (I.V.) selon la revendication 1, dans lequel la boule (8) est plus large que la circonférence externe du trou centrique de la rondelle numéro I (6) et du trou centrique de la rondelle numéro II (7).

6. Dispositif de cathéter intraveineux (I.V.) selon la revendication 1, dans lequel la boule (8) comprend l'un de : l'acier inoxydable, le titane, ou du plastique.

7. Dispositif de cathéter intraveineux (I.V.) selon la revendication 1, dans lequel l'aiguille comprend de l'acier inoxydable.

8. Dispositif de cathéter intraveineux (I.V.) selon la revendication 1, dans lequel le corps (2) et/ou le pavillon d'aiguille (5) du cathéter (I.V.) comprend du polypropylène.
